# EUROPEAN PATENT APPLICATION

(11) **EP 4 082 526 A1**
(43) Date of publication of application: **02.11.2022**
(21) Application number: 21305567.6
(22) Date of filing: 30.04.2021
(51) Int. Cl.: A61K 9/00, A61K 6/00, A61K 9/08, A61K 31/167, A61K 47/10, A61K 47/18, A61K 47/22, A61P 23/02

(54) **INJECTABLE ANESTHETIC SOLUTION WITH A REDUCED BITTERNESS**

(71) Applicant: Septodont ou Septodont SAS ou Specialites Septodont, 94100 Saint Maur des Fossés (FR)
(72) Inventor: RICHARD, Gilles, 91560 Crosnes (FR); PISANI, Emilia, 75011 Paris (FR); BALESTRA, Richard, 91320 Wissous (FR); ARTAUD, Laurent, 94340 Joinville-Le-Pont (FR)
(74) Representative: Icosa

(57) **Abstract**

The present invention relates to the field of anesthetics. Especially, the present invention refers to an injectable anesthetic solution having a reduced bitterness comprising:
- an anesthetic agent;
- optionally, a vasoconstrictor;
- a pharmaceutically acceptable solvent; and
- a bitterness suppressant comprising or consisting of a mixture of at least two among sodium saccharin, sorbitol and an amino acid, wherein the amino acid is selected from: serine, threonine and their mixtures.

The present invention also relates to a method for providing said injectable anesthetic solution and its uses in dentistry, oral surgery and/or maxillofacial surgery.

## Description

### FIELD OF INVENTION

The present invention relates to the field of anesthetics. Especially, the present invention refers to an injectable anesthetic solution having a reduced bitterness, comprising a local anesthetic, optionally a vasoconstrictor, a pharmaceutically acceptable solvent and a bitterness suppressant including a sweetener selected from saccharin and/or sorbitol; and/or an amino acid selected from serin and/or threonine. The present invention also relates to the method for manufacturing the injectable anesthetic solution and its uses in dentistry, oral surgery and/or maxillofacial surgery.

### BACKGROUND OF INVENTION

In recent years, dental procedures such as the number of fillings, root canals, crowns, and extractions increased significantly. Most treatments require injecting local anesthetics such as lidocaine-based anesthetics, in order to guarantee high patient comfort.

During the administration of the anesthetics, part of the injected volume may reflux back onto the oral cavity and tongue due to resistance from patients and improper injection techniques, resulting in patients experiencing the bitter and unpalatable taste of the local oral anesthetics. This experience is not well-received by patients, in particular by children. Furthermore, the bitter taste in the mouth of the patient may last up to two hours after the dissipation of the anesthetic.

Thus, eliminating the bitter taste of dental products would partially enhance patient tolerance, change the patient's perspective towards the dental procedures, and render oral care visits more pleasant.

Limited attempts have been made to mask the taste of dental products. The main reported techniques to reduce the bitterness of oral anesthetics deal with providing a coating with insoluble polymers, adding sweeteners or flavors, using ion exchange resins, complexing with cyclodextrins and/or using prodrugs. In these techniques, the aim is to limit or avoid contacting the anesthetics with the taste buds. However, the main drawback of these techniques is that they cannot be implemented in an injectable formulation due to the stringent requirements and policies of the Food and Drug Administration (FDA). Indeed, the compounds of the injectable formulation have to comply with the injection requirements such as for example a suitable osmolarity. However, until now, the addition of taste masking agents in an injectable formulation causes a modification of its osmolarity, in particular an over osmolarity, rendering them incompatible for injection.

Furthermore, the addition of compounds in the injectable solution should not alter the chemical stability of the active agents.

Consequently, reducing or eliminating the bitter taste of oral injectable anesthetics is still challenging. Thus, there is still a need for providing injectable anesthetics overcoming these drawbacks.

### SUMMARY

The present invention refers to an injectable anesthetic solution comprising:
- an anesthetic agent, preferably selected from lidocaine derivates; tetracaine derivates; xylocaine derivates; mepivacaine derivates; prilocaine derivates; bupivacaine derivates; etidocaine derivates; ropivacaine derivates; articaine derivates; and any combinations thereof;
- optionally, a vasoconstrictor;
- a pharmaceutically acceptable solvent; and
- a bitterness suppressant comprising or consisting of a mixture of at least two among sodium saccharin, sorbitol and an amino acid, wherein the amino acid is selected from: serine, threonine and their mixtures.

According to one embodiment, the injectable anesthetic solution comprises a vasoconstrictor which is an epinephrin, preferably the vasoconstrictor is epinephrin bitartrate.

According to one embodiment, the anesthetic agent is a lidocaine or a derivate thereof; preferably is lidocaine hydrochloride.

According to one embodiment, the pharmaceutically acceptable solvent is water, preferably selected from sterilized water, purified water and osmosed water.

According to one embodiment, the bitterness suppressant consists of sodium saccharin and serine.

According to one embodiment, the amount of amino acid ranges from 1% to 6% wt., preferably ranges from 2% to 3% wt., relative to the total weight of the injectable anesthetic solution.

According to one embodiment, the amount of sodium saccharin and/or sorbitol, preferably sodium saccharin, ranges from 0.01% to 5% wt., preferably from 0.01% to 1% wt., more preferably is 0.09% relative to the total weight of the injectable anesthetic solution.

According to one embodiment, the injectable anesthetic solution of the invention further comprises one or more additives selected from the group consisting of: buffering agents; preservative compounds; stabilizers; contrast media agents; and their mixtures thereof.

According to one embodiment, the preservative compound is selected from: sodium bisulfites, potassium bisulfites or metabisulfites, ascorbic acid, citric acid, ethylene diamine tetra acetic acid (EDTA) and their salts ("edetate salts") such as disodium edetate, sodium hydroxide, benzyl alcohol, phenylethyl alcohol, phenol, meta-cresol, chlorobutanol, thimerosal, phenylmercuric salts, and any combinations thereof; preferably is sodium metabisulfite or potassium metabisulfite; more preferably the injectable anesthetic solution comprises a preservative compound which is selected from disodium edetate, sodium hydroxide and any mixture thereof. According to one embodiment, the injectable anesthetic solution does not comprise ethylene diamine tetra acetic acid (EDTA).

According to one embodiment, the injectable anesthetic solution comprises:
- from 0.01% wt. to 5% wt., preferably 2.1% wt., of an anesthetic agent which is lidocaine hydrochloride;
- from 0.0001% wt. to 1% wt., preferably 0.002% wt., of a vasoconstrictor which is epinephrin bitartrate;
- a pharmaceutically acceptable solvent; and
- a bitterness suppressant comprising or consisting of:
   (i) from 0.01% to 5% wt., preferably 0.09%, 3% or 3.09%, of a sweetener selected from: sodium saccharin, sorbitol and their mixtures; and
   (ii) from 1% to 6% wt., preferably from 2% to 3% wt., more preferably is 2.4% or 2.7%, of an amino acid selected from: serine, threonine and their mixtures;
   said amounts being relative to the total weight of the injectable anesthetic solution.

The present invention also refers to a method for manufacturing the injectable anesthetic solution of the invention, comprising mixing:
- an anesthetic agent, preferably which is lidocaine or a derivative thereof;
- optionally, a vasoconstrictor, preferably which is epinephrin bitartrate;
- a pharmaceutically acceptable solvent; and
- a bitterness suppressant comprising or consisting of a mixture of at least two among sodium saccharin, sorbitol and an amino acid, wherein the amino acid is selected from: serine, threonine and their mixtures.

According to one embodiment, the injectable anesthetic solution according of the invention is for use in anesthesia during dentistry, oral surgery and/or maxillofacial surgery.

According to one embodiment, dentistry, oral surgery and/or maxillofacial surgery is filling dental root canals.

According to one embodiment, dentistry, oral surgery and/or maxillofacial surgery is dental crowns positioning and/or root extractions.

The present invention also refers to a pre-filled syringe filled with the injectable anesthetic solution of the invention.

### DEFINITIONS

In the present invention, the following terms have the following meanings:
- **"About":** preceding a figure means plus or less 10%, preferably plus or less 5%, more preferably plus or less 1%, of the value of said figure.
- **"Amino acid":** refers to any chemical compound having a basic amino group (-NH₂), an acidic carboxyl group (-COOH) and an organic group (or side chain) that is unique to each amino acid. According to one embodiment, the amino acid is selected from nonpolar amino acids such as glycine, alanine, valine, leucine, isoleucine, proline, phenylalanine, methionine and tryptophan; polar and uncharged amino acids such as serine, cysteine, threonine, tyrosine, asparagine and glutamine; acidic amino acids such as aspartic acid and glutamic acid; and basic amino acids such as arginine, histidine and lysine.
- **"Anesthetic"** or **"anaesthetic"** refers to any drug inducing anesthesia when administered to a patient. According to one embodiment, the terms **"Anesthetic"** and **"anaesthetic"** refer to any drug inducing a temporary loss of sensation and/or awareness in a patient. According to one embodiment, the anesthetic is a local anesthetic, i.e. a drug causing a reversible loss of sensation in a limited area of the body of the patient. According to one embodiment, the local anesthetic in the present invention is a drug causing a reversible loss of sensation of one or more teeth, of the lower and/or upper jaws, and/or of the tongue of the patient.
- **"Bisulfite"** or **"hydrogen sulfite":** refers to any chemical compound comprising the anion HSO₃⁻. **"Metabisulfite":** refers to any chemical compound comprising the anion S₂O₅²⁻.
- **"Bitterness suppressant":** refers to any compound or association of compounds that when added into a composition, lowers or avoids the bitterness of said composition compared to its taste without said bitterness suppressant.
- **"Buffering agent":** refers to any compound used to adjust and/or maintain the pH in a composition.
- **"Contrast media agent":** refers to any chemical agents that help in the characterization of a pathology by improving the contrast resolution of an imaging modality.
- **"Effective amount":** refers to the amount of an anesthetic necessary and sufficient for providing an anesthetic effect.
- **"Injectable":** refers to any compound or composition suitable to be administered to a patient with a device having a needle or a catheter. According to one embodiment, "injectable" refers to a solution suitable to be administered with a syringe to a patient.
- **"Lidocaine"** or **"2-(diethylamino)-N-(2,6-dimethylphenyl)acetamide":** refers to a local anesthetic belonging to the group of amino-amides and having the following formula (I): 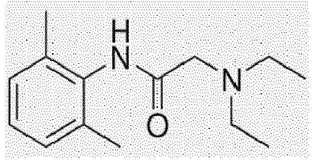 According to one embodiment, the terms "lidocaine derivates" refer to any compounds having the backbone of the lidocaine formula as described above or a similar backbone, and optionally substituted by one or more chemical groups identical or different from the substituents of lidocaine. According to one embodiment, the terms "lidocaine derivates" include the formula of the lidocaine. According to one embodiment, the terms "lidocaine derivates" refer to any compounds having the following formula (II): 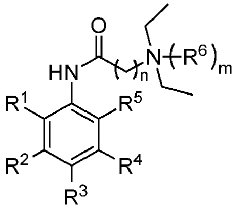 wherein:
   n refers to a positive figure; preferably n equals 0, 1 or 2;
   m equals 0 or 1; and
   R¹, R², R³, R⁴, R⁵ and R⁶ each independently, represent H, alkyl, alkoxyl, halogenoalkyl such as fluoroalkyl, or hydroxyl; said group being optionally substituted by an aryl.
   When m equals 1, the nitrogen atom is under the form of a quaternary ammonium (and thus has a positive charge). According to one embodiment, when m equals 1, the compound of formula (II) is in presence of a counter-ion. According to one embodiment, when m equals 0, there is no R⁶ group in formula (II). According to one embodiment, m is 0, n is 1, R¹ and R⁵ are methyl, and R², R³ and R⁴ are H. According to one embodiment, m is 1, n is 1, R¹ and R⁵ are methyl, and R², R³ and R⁴ are H; and R⁶ is selected from an ethyl group which is not substituted, a dodecyl group or an ethyl group substituted by a phenyl. The term **"derivates"** applies similarly to other anesthetic agents such as tetracaine; xylocaine; mepivacaine; prilocaine; bupivacaine; etidocaine; ropivacaine; or articaine.
- **"Parabens"** or **"paraben derivatives"** refers to a family of compounds having as backbone, the chemical structure of *para*-hydroxybenzoate; said chemical structure may be substituted or not by one or more alkyl groups. According to one embodiment, the paraben derivatives may be methylparaben, ethylparaben, propylparaben or butylparaben.
- **"Pharmaceutically acceptable salt"** refers to any salts which are non-toxic and have no significant impurities or degradation products formed as a result of chemical breakdown of the salt, alone or in combination with excipients and which are suitable for making a dosage form that is administrable to a patient. According to one embodiment, the pharmaceutically acceptable salt may be hydrochloride or tartrate, preferably bitartrate.
- **"Preservative compound"** or **"stabilizer":** refers to any chemical compound added in a composition for protecting it against degradation.
- **"Vasoconstrictor":** refers to any drug inducing vasoconstriction.
- **"Saccharin":** refers to a compound having as backbone: 1,1-dioxo-1,2-benzothiazol-3-one. According to one embodiment, the saccharin is sodium saccharin.
- **"Serin":** refers to 2-amino-3-hydroxypropanoic acid.
- **"Threonine":** refers to 2-amino-3-hydroxybutanoic acid.

### DETAILED DESCRIPTION

### Injectable anesthetic solution

This invention relates to an anesthetic composition, preferably an anesthetic liquid composition, more preferably an anesthetic solution. According to one embodiment, the anesthetic solution is an injectable anesthetic solution. According to one embodiment, the injectable anesthetic solution comprises or consists of:
- an anesthetic agent;
- optionally, a vasoconstrictor;
- a pharmaceutically acceptable solvent; and
- a bitterness suppressant.

According to one embodiment, the injectable anesthetic solution comprises or consists of:
- an anesthetic agent;
- a vasoconstrictor;
- a pharmaceutically acceptable solvent; and
- a bitterness suppressant.

### Anesthetic agent

According to one embodiment, the anesthetic agent is a local anesthetic. According to one embodiment, the anesthetic agent is an injectable anesthetic. According to one embodiment, the anesthetic agent is an injectable local anesthetic.

According to one embodiment, the anesthetic agent is selected from: lidocaine derivates; tetracaine derivates; xylocaine derivates; mepivacaine derivates; prilocaine derivates; bupivacaine derivates; etidocaine derivates; ropivacaine derivates; articaine derivates; and any combinations thereof. According to one embodiment, the anesthetic agent is lidocaine or its derivates. According to one embodiment, the anesthetic agent is lidocaine hydrochloride. In the present invention, the term "X derivates" where X is the name of an anesthetic agent, includes the anesthetic agent X itself and any chemical substitutions made on the backbone of said anesthetic agent X or any pharmaceutically acceptable salts of said anesthetic agent X.

According to one embodiment, the anesthetic agent is in an amount ranging from 0.01% wt. to 10% wt., preferably from 1% wt. to 5% wt., more preferably from 1% to 3% wt., even more preferably is 2% wt. or 2.1% wt., relative to the total weight of the injectable anesthetic solution. According to one embodiment, the anesthetic agent is in an amount of 0.01%; 0.02%; 0.03%; 0.04%; 0.05%; 0.06%; 0.07%; 0.08%; 0.09%; 0.1%; 0.2%; 0.3%; 0.4%; 0.5%; 0.6%; 0.7%; 0.8%; 0.9%; 1%; 1.1%; 1.2%; 1.3%; 1.4%; 1.5%; 1.6%; 1.7%; 1.8%; 1.9%; 2%; 2.1%; 2.2%; 2.3%; 2.4%; 2.5%; 2.6%; 2.7%; 2.8%; 2.9%; 3%; 3.1%; 3.2%; 3.3%; 3.4%; 3.5%; 3.6%; 3.7%; 3.8%; 3.9%; 4%; 4.1%; 4.2%; 4.3%; 4.4%; 4.5%; 4.6%; 4.7%; 4.8%; 4.9% or 5% wt., relative to the total weight of the injectable anesthetic solution.

### Vasoconstrictor

According to one embodiment, the vasoconstrictor is selected from: adrenaline; epinephrin; norepinephrin; phenylephrine; felypressin; levonordefrin or any pharmaceutically acceptable salt thereof; and any combinations thereof. According to one embodiment, the vasoconstrictor is a catecholamine; preferably adrenalin, epinephrin or norepinephrin; more preferably epinephrin; even more preferably epinephrin bitartrate. According to one embodiment, the vasoconstrictor is in an amount ranging from 0.0001% wt. to 5% wt., preferably from 0.0001% to 1% wt., more preferably in an amount of 0.002% wt., relative to the total weight of the injectable anesthetic solution. According to one embodiment, the anesthetic agent is in an amount of 0.001%; 0.002%; 0.003%; 0.004%; 0.005%; 0.006%; 0.007%; 0.008%; 0.009%; 0.01%; 0.02%; 0.03%; 0.04%; 0.05%; 0.06%; 0.07%; 0.08%; 0.09%; 0.1%; 0.2%; 0.3%; 0.4%; 0.5%; 0.6%; 0.7%; 0.8%; 0.9%; or 1% wt., relative to the total weight of the injectable anesthetic solution.

### Pharmaceutically acceptable solvent

According to one embodiment, the pharmaceutically acceptable solvent is water; preferably the pharmaceutically acceptable solvent is sterilized water, purified water or osmosed water.

According to one embodiment, the amount of pharmaceutically acceptable solvent in the injectable anesthetic solution ranges from 50% wt. to 98% wt.; preferably from 70% wt. to 97% wt.; more preferably from 80% wt. to 96% wt.; even more preferably is 95.6% wt. relative to the total weight of said injectable anesthetic solution. According to one embodiment, the amount of the pharmaceutically acceptable solvent in the injectable anesthetic solution is 95%, 95.1%, 95.2%, 95.3%, 95.4%, 95.5%, 95.6%, 95.7%, 95.8%, 95.9%, 96%, 96.1%, 96.2%, 96.3%, 96.4%, 96.5%, 96.6%, 96.7%, 96.8%, 96.9%, 97%, 97.1%, 97.2%, 97.3%, 97.4%, 97.5%, 97.6%, 97.7%, 97.8%, 97.9% or 98% wt., relative to the total weight of said injectable anesthetic solution.

### Bitterness suppressant

According to one embodiment, the bitterness suppressant comprises or consists of at least one sweetener and/or at least one amino acid. According to one embodiment, the bitterness suppressant consists of a sweetener and an amino acid. According to one embodiment, the bitterness suppressant consists of a mixture of sweeteners.

According to one embodiment, the sweetener is an artificial sweetener; preferably is a saccharin; more preferably is sodium saccharin. According to one embodiment, the sweetener is a natural sweetener; preferably is sorbitol (CAS 50-70-4). According to one embodiment, the sweetener is selected from saccharin, sorbitol and their mixtures.

According to one embodiment, the amino acid is selected from nonpolar amino acids such as glycine, alanine, valine, leucine, isoleucine, proline, phenylalanine, methionine and tryptophan; polar and uncharged amino acids such as serine, cysteine, threonine, tyrosine, asparagine and glutamine; acidic amino acids such as aspartic acid and glutamic acid; and basic amino acids such as arginine, histidine and lysine. According to one embodiment, the amino acid is serine and/or threonine. According to one embodiment, the amino acid is serine. According to one embodiment, the amino acid is threonine.

According to one embodiment, the bitterness suppressant comprises or consists of sodium saccharin and an amino acid selected from glycine, alanine, valine, leucine, isoleucine, proline, phenylalanine, methionine and tryptophan, serine, cysteine, threonine, tyrosine, asparagine and glutamine, aspartic acid, glutamic acid, arginine, histidine and lysine. According to one embodiment, the bitterness suppressant comprises or consists of sodium saccharin and serine. According to one embodiment, the bitterness suppressant comprises or consists of sodium saccharin and threonine. According to one embodiment, the bitterness suppressant consists of sodium saccharin, serin and threonine. According to one embodiment, the bitterness suppressant consists of sodium saccharin and sorbitol. According to one embodiment, the bitterness suppressant consists of sodium saccharin, sorbitol and serin. According to one embodiment, the bitterness suppressant consists of sodium saccharin, sorbitol and threonine. According to one embodiment, the bitterness suppressant consists of sorbitol and threonine. According to one embodiment, the bitterness suppressant consists of sorbitol, serin and threonine.

According to one embodiment, the amount of bitterness suppressant in the injectable anesthetic solution ranges from 1% wt. to 6% wt., preferably from 1.5% wt. to 3% wt., more preferably is 2.09% wt., relative to the total weight of said injectable anesthetic solution. According to one embodiment, the amount of the bitterness suppressant in the injectable anesthetic solution is 1%, 2%, 3%, 4%, 5% or 6% wt., relative to the total weight of said injectable anesthetic solution.

According to one embodiment, the amount of sweetener in the injectable anesthetic solution ranges from 0.01% to 5% wt., preferably from 0.01% wt. to 1% wt., more preferably from 0.01% to 0.1%, even more preferably is 0.09% wt., relative to the total weight of said injectable anesthetic solution. According to one embodiment, the amount of sweetener in the injectable anesthetic solution is 0.01%; 0.02%; 0.03%; 0.04%; 0.05%; 0.06%; 0.07%; 0.08%; 0.09%; 0.1%; 0.2%; 0.3%; 0.4%; 0.5%; 0.6%; 0.7%; 0.8%; 0.9%; or 1% wt., relative to the total weight of the injectable anesthetic solution. According to one embodiment, the amount of sweetener in the injectable anesthetic solution is 1%; 2%; 3%; 4% or 5% wt., relative to the total weight of the injectable anesthetic solution.

According to one embodiment, the injectable anesthetic solution comprises:
- from 0.1% to 4% wt., preferably 1%, 2% or 3% wt., of sorbitol; and
- from 0.01% to 1% wt., preferably 0.09% wt., of sodium saccharin.

According to one embodiment, the amount of amino acid in the injectable anesthetic solution ranges from 0.1% wt. to 5% wt., preferably from 1% to 4%, more preferably is 2% wt. or 3%. wt., relative to the total weight of said injectable anesthetic solution. According to one embodiment, the amount of the amino acid in the injectable anesthetic solution is 0.1%; 0.2%; 0.3%; 0.4%; 0.5%; 0.6%; 0.7%; 0.8%; 0.9%; or 1% wt., relative to the total weight of the injectable anesthetic solution.

### Additives

According to one embodiment, the injectable anesthetic solution further comprises one or more additives. According to one embodiment, the additive is selected from the group consisting of: buffering agents; preservative compounds or stabilizers; contrast media agents; chelating agents such as ethylenediaminetetraacetic acid (EDTA), citric acid, ascorbic acid; osmotic agents; and any mixture thereof.

According to one embodiment, the buffering agent is selected from alkaline, neutral or acid substances of sodium bicarbonate; Hartmann's solution; Ringer's solution; lactated Ringer's solution; acetated Ringer's solution; bicarbonated Ringer's solution; colloids-based agents; and any combinations thereof.

According to one embodiment, the preservative compound or the stabilizer is selected from: paraben derivatives, bisulfites or metabisulfites, compounds comprising one or more thiol functions such as acetylcysteine, cysteine, or thioglycerol, and any combinations thereof. According to one embodiment, the preservative compound is a metabisulfite, preferably is potassium metabisulfite.

According to one embodiment, the amount of the preservative compound in the injectable anesthetic solution ranges from 0% wt. to 1% wt., preferably from 0.01% to 0.5%, more preferably is 0.12 % wt., to the total weight of said the injectable anesthetic solution.

According to one embodiment, the contrast media agent is selected from silver-based agents; barium- based agents; ionic iodine; non-ionic iodine; sodium iodide, sodium iothalamate, amino sugar derivates; amino sugar derivates combined with iodinated compounds; gadolinium-based agents; iron; iron oxide; iron platinum; manganese; perflubron; nitrogen; perfluorocarbon; protein-based and pharmaceutical prepared microbubble contrast media; and any combinations thereof.

### Specific solutions

According to one embodiment, the injectable anesthetic solution comprises or consists of:
- from 0.01% wt. to 5% wt. of an anesthetic agent;
- from 0.001% wt. to 1% wt. of a vasoconstrictor;
- a pharmaceutically acceptable solvent; and
- from 1% wt. to 6% wt. of a bitterness suppressant;
   said amounts being relative to the total weight of the injectable anesthetic solution.

According to one embodiment, the injectable anesthetic solution comprises or consists of:
- from 0.01% wt. to 5% wt. of an anesthetic agent; preferably selected from lidocaine derivates; tetracaine derivates; xylocaine derivates; mepivacaine derivates; prilocaine derivates; bupivacaine derivates; etidocaine derivates; ropivacaine derivates; articaine derivates; and any combinations thereof;
- from 0.001% wt. to 1% wt. of a vasoconstrictor;
- a pharmaceutically acceptable solvent; and
- a bitterness suppressant comprising or consisting of:
   (i) from 0.01% to 1% wt., preferably 0.09%, of a sweetener selected from: sodium saccharin, sorbitol and their mixtures; and
   (ii) from 1% to 5% wt., preferably 2%, of an amino acid selected from: serine, threonine and their mixtures;
   said amounts being relative to the total weight of the injectable anesthetic solution.

According to one embodiment, the injectable anesthetic solution comprises or consists of:
- from 0.01% wt. to 5% wt. of an anesthetic agent; preferably selected from lidocaine derivates; tetracaine derivates; xylocaine derivates; mepivacaine derivates; prilocaine derivates; bupivacaine derivates; etidocaine derivates; ropivacaine derivates; articaine derivates; and any combinations thereof;
- from 0.001% wt. to 1% wt. of a vasoconstrictor;
- a pharmaceutically acceptable solvent; and
- a bitterness suppressant comprising or consisting of: from 0.01% to 5% wt. of a mixture of sodium saccharin and sorbitol;
   said amounts being relative to the total weight of the injectable anesthetic solution.

According to one embodiment, the injectable anesthetic solution comprises or consists of:
- from 0.01% wt. to 5% wt. of an anesthetic agent; preferably selected from lidocaine derivates; tetracaine derivates; xylocaine derivates; mepivacaine derivates; prilocaine derivates; bupivacaine derivates; etidocaine derivates; ropivacaine derivates; articaine derivates; and any combinations thereof;
- from 0.001% wt. to 1% wt. of a vasoconstrictor;
- a pharmaceutically acceptable solvent; and
- a bitterness suppressant comprising or consisting of:
   from 0.01% to 1% wt., preferably 0.09% wt., of sodium saccharin and
   from 0.01% to 4%, preferably 3% wt., of sorbitol;
   said amounts being relative to the total weight of the injectable anesthetic solution.

According to one embodiment, the injectable anesthetic solution comprises or consists of:
- from 0.01% wt. to 5% wt. of an anesthetic agent; preferably selected from lidocaine derivates; tetracaine derivates; xylocaine derivates; mepivacaine derivates; prilocaine derivates; bupivacaine derivates; etidocaine derivates; ropivacaine derivates; articaine derivates; and any combinations thereof;
- from 0.001% wt. to 1% wt. of a vasoconstrictor;
- a pharmaceutically acceptable solvent; and
- a bitterness suppressant comprising or consisting of:
   (i) from 0.01% to 1% wt., preferably 0.09%, of sodium saccharin; and
   (ii) from 1% to 5% wt., preferably 2%, of an amino acid selected from: serine, threonine and their mixtures;
   said amounts being relative to the total weight of the injectable anesthetic solution.

According to one embodiment, the injectable anesthetic solution comprises or consists of:
- from 0.01% wt. to 5% wt. of lidocaine or its derivates, preferably lidocaine hydrochloride;
- from 0.001% wt. to 1% wt. of a catecholamine, preferably epinephrin, more preferably epinephrin bitartrate;
- a pharmaceutically acceptable solvent; and
- a bitterness suppressant comprising or consisting of:
   (i) from 0.01% to 1% wt., preferably 0.09%, of sodium saccharin; and
   (ii) from 1% to 5% wt., preferably 2%, of an amino acid selected from: serine, threonine and their mixtures;
   said amounts being relative to the total weight of the injectable anesthetic solution.

According to one embodiment, the injectable anesthetic solution comprises or consists of:
- about 2.1% wt. of lidocaine or its derivates, preferably lidocaine hydrochloride;
- about 0.002% wt. of a catecholamine, preferably epinephrin, more preferably epinephrin bitartrate;
- a pharmaceutically acceptable solvent; and
- a bitterness suppressant comprising or consisting of:
   (i) from 0.01% to 1% wt., preferably 0.09%, of sweetener selected from: sodium saccharin, sorbitol and their mixtures; and
   (ii) from 1% to 5% wt., preferably 2%, of an amino acid selected from: serine, threonine and their mixtures;
   said amounts being relative to the total weight of the injectable anesthetic solution.

According to one embodiment, the injectable anesthetic solution comprises or consists of:
- about 2.1% wt. of lidocaine or its derivates, preferably lidocaine hydrochloride;
- about 0.002% wt. of a catecholamine, preferably epinephrin, more preferably epinephrin bitartrate;
- a pharmaceutically acceptable solvent; and
- a bitterness suppressant comprising or consisting of:
   (i) from 0.01% to 1% wt., preferably 0.09%, of sodium saccharin; and
   (ii) from 1% to 5% wt., preferably 2%, of an amino acid selected from: serine, threonine and their mixtures;
   said amounts being relative to the total weight of the injectable anesthetic solution.

The present description is written in connection with an injectable solution but any technical feature applies mutatis mutandis to other possible forms for the anesthetic composition.

### Method for manufacturing the anesthetic composition

The invention also relates to a method for manufacturing an anesthetic composition, preferably for manufacturing an injectable anesthetic solution.

According to one embodiment, the method of the invention comprises or consists of mixing:
- an anesthetic agent as defined above;
- optionally, a vasoconstrictor as defined above;
- a pharmaceutically acceptable solvent as defined above; and
- a bitterness suppressant as defined above.

The method for manufacturing the anesthetic composition of the invention may be implemented by any suitable method known in the art.

Mixing steps may for instance be implemented by mechanical stirring.

The different elements to be mixed for manufacturing the anesthetic composition according to the invention may be mixed in any order.

### Injection device and syringe

The invention also relates to an injection device comprising an injectable anesthetic solution as defined above. According to one embodiment, the injection device is a syringe, preferably a monosyringe; more preferably a pre-filled syringe with the injectable anesthetic solution as defined above.

### Uses & Method for reducing bitterness

The invention also relates to the use of the injectable anesthetic solution as defined above. According to one embodiment, the injectable anesthetic solution as defined above is useful in dentistry, oral surgery and/or maxillofacial surgery. According to one embodiment, the injectable anesthetic solution as defined above is useful for reducing pain in a patient. According to one embodiment, the injectable anesthetic solution as defined above has a reduced bitterness compared to an injectable anesthetic solution that does not comprise the bitterness suppressant of the present invention.

According to one embodiment, the present invention also relates to a method for reducing the bitterness of an anesthetic composition, preferably of an anesthetic solution, more preferably of an injectable anesthetic solution. According to one embodiment, the present invention also relates to a method for reducing the bitterness felt by a patient when an injectable anesthetic solution is administered to him/her. Preferably, the anesthetic composition which bitterness is reduced with the method of the invention comprises an anesthetic agent, preferably selected from lidocaine derivates; tetracaine derivates; xylocaine derivates; mepivacaine derivates; prilocaine derivates; bupivacaine derivates; etidocaine derivates; ropivacaine derivates; articaine derivates; and any combinations thereof, optionally a vasoconstrictor, and a pharmaceutically acceptable solvent.

According to one embodiment, the method for reducing the bitterness of an anesthetic composition comprises the addition to said anesthetic composition of a sweetener and/or an amino acid, preferably the addition of saccharin and an amino acid selected from serine and/or threonine, more preferably the addition of sodium saccharin and an amino acid selected from serine and/or threonine. According to one embodiment, the method for reducing the bitterness of an anesthetic composition comprises the addition to said anesthetic composition of 0.09% wt. of sodium saccharin and 2% wt. or 3% wt. of an amino acid selected from serine and/or threonine.

The invention also relates to a method for anesthetizing a part of or the whole oral cavity of a subject during dentistry, oral surgery and/or maxillofacial surgery, comprising administering an effective amount of an injectable anesthetic solution of the invention to said subject., preferably by injection, in particular implemented with an injection device of the invention. According to one embodiment, the injectable anesthetic solution is administered in a part of the oral cavity. According to one embodiment, the part of the oral cavity may be selected from: one or more teeth, hard palate, soft palate, tongue, uvula, floor of mouth, gums, retromolar trigone, tonsil and buccal mucosa.

According to one embodiment, the method for anesthetizing a part of or the whole oral cavity of a subject is implemented during filling dental root canals.

According to one embodiment, the method for anesthetizing a part of or the whole oral cavity of a subject is implemented during dental crowns positioning and/or root extractions.

### BRIEF DESCRIPTION OF THE DRAWINGS

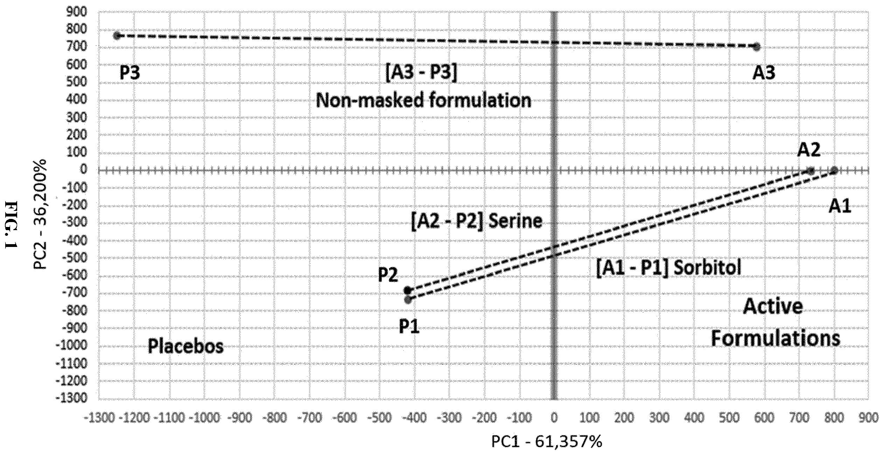

**Figure 1** is a taste map obtained by using an Astree e-tongue system with two injectable anesthetic solutions of the invention (A1 and A2), their corresponding placebos (P1 and P2), and the corresponding non-masked formulations (A3: formulation without any active agents and P3: the corresponding placebo).

### EXAMPLES

The present invention is further illustrated by the following examples.

### Example 1: Examples of injectable anesthetic solutions of the invention

Several solutions according to the present invention have been prepared:

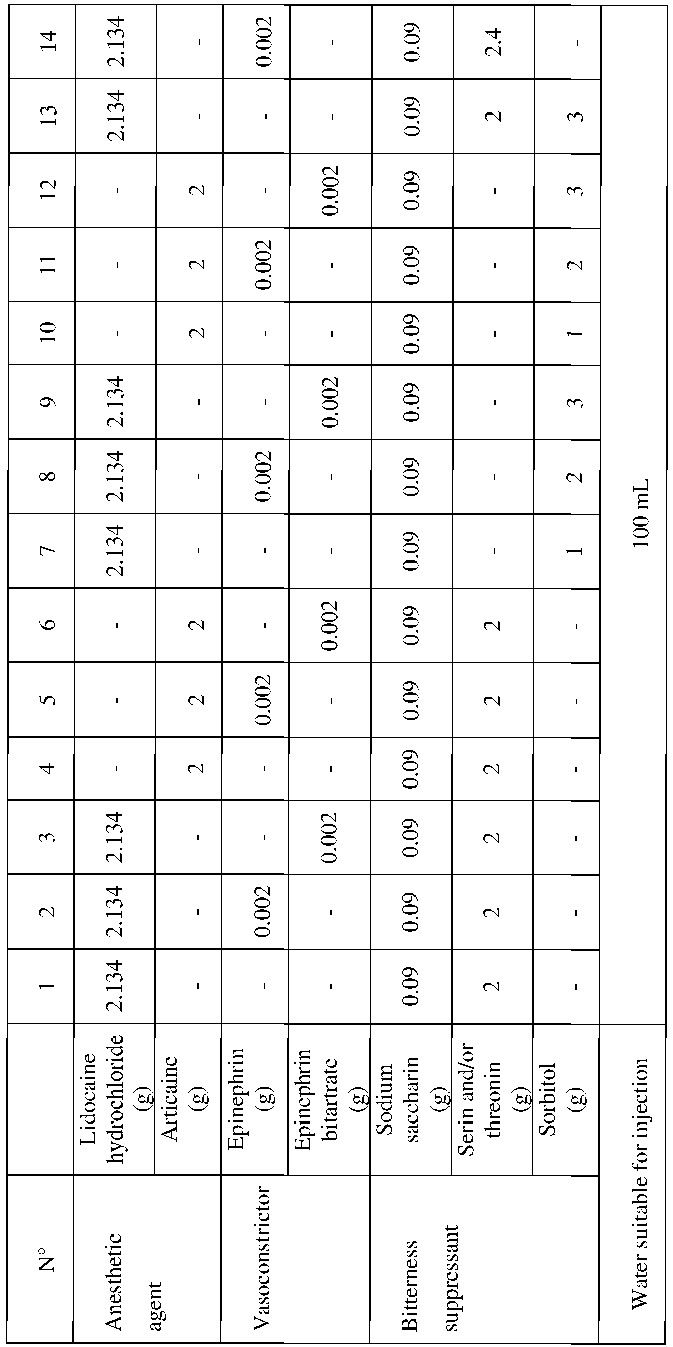

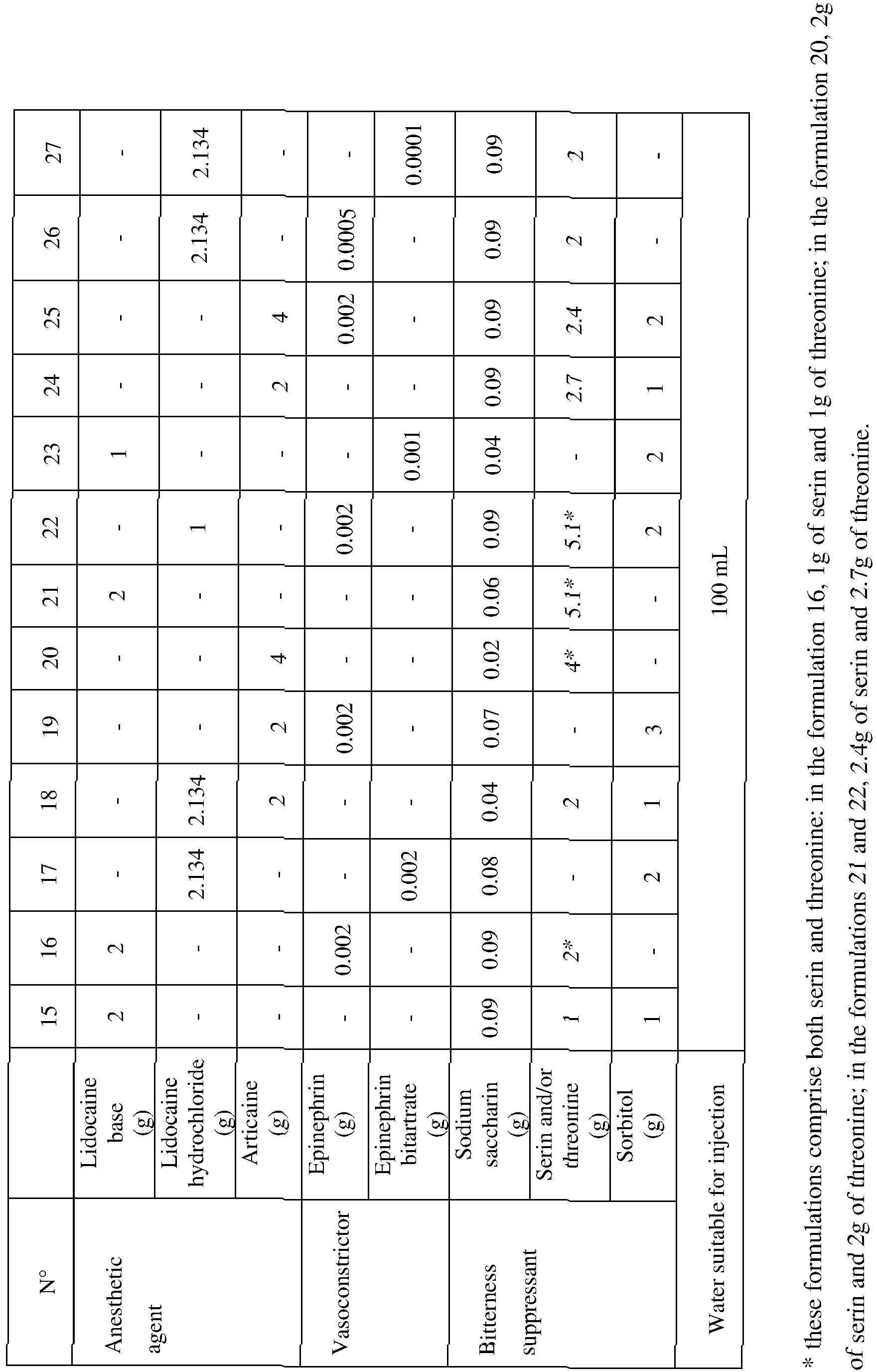

In this Table, the quantity of the components is given for 100 mL of the injectable solution.

According to the present invention, the lidocaine hydrochloride or the articaine used in the formulations 1-25 of the above table may be replaced by any anesthetic agent known by the skilled artisan, such as for example: any derivates of lidocaine, any derivates of articaine, tetracaine derivates; xylocaine derivates; mepivacaine derivates; prilocaine derivates; bupivacaine derivates; etidocaine derivates; ropivacaine derivates; and any combinations thereof.

### Example 2: Assessment of the bitterness reduction with the solution of the invention

The aim is to compare the taste of the injectable anesthetic solution of the invention with that of the corresponding non-masked taste formulation; and to determine if the bitterness of the injectable anesthetic solution of the invention is reduced.

For this goal, a taste comparison was carried out between the injectable anesthetic solution of the invention A1 (i.e. solution of lidocaine hydrochloride and epinephrin bitartrate including a bitterness suppressant consisting of 0.09% wt. of sodium saccharine and 3% wt. of sorbitol, by the total weight of said injectable anesthetic solution); the injectable anesthetic solution of the invention A2 (i.e. solution of lidocaine hydrochloride and epinephrin bitartrate including a bitterness suppressant consisting of 0.09% wt. of sodium saccharine and 2% wt. of serin, by the total weight of said injectable anesthetic solution); their corresponding placebos (P1 and P2, i.e. without lidocaine hydrochloride) and the corresponding non-masked formulations (P3: non-masked placebo and A3: non-masked active formulation).

The masking effect of the injectable anesthetic solutions is estimated by the determination of the distances between a e-tongue signal of said formulation containing the active ingredients and the formulations without lidocaine hydrochloride (placebo). The best masking formulation is the one giving the optimum distance.

### Material & Methods

The assays were realized on Astree e-tongue system equipped with an Alpha M.O.S. sensor set composed of 7 specific sensors (AHS, PKS, CTS, NMS, CPS, ANS, SCS) on a 48-positions autosampler using 25 ml-beakers. Acquisition times were fixed at 120s during 180 s per analysis. All the data generated on Astree system were treated using multidimensional statistics on AlphaSoft V15 software.

The e-tongue signal in each sample was measured at the equilibrium on 7 sensors (average between 100 and 120s). Three replicates were taken into account for the analysis.

### Results

The Euclidian distances between placebos and formulations were calculated to assess taste proximity between samples: the lower the distance, the closer the taste. The results are presented in the taste map of Figure 1.

The taste map of Figure 1 shows that:
- The distance between A3 and P3 is the longest so the bitterness is caused by the lidocaine hydrochloride;
- When the formulations comprise the bitterness suppressant (i.e. sodium saccharin combined either to sorbitol or to serin), the distance between A1 and P1 and the distance between A2 and P2 are shorter than the distance between A3 and P3, thus, the bitterness is lower in the formulations comprising the bitterness suppressant.

In conclusion, the addition of 0.09%wt. of sodium saccharin with either 2% wt. of serine or 3% wt. of sorbitol in an injectable solution of lidocaine hydrochloride and epinephrin bitartrate allows reducing its bitterness compared to non-masked taste formulations. Compared to non-masked formulations, the addition of sodium saccharin with either sorbitol or serin reduces the bitterness respectively of 21% and 26%.

### Example 3: Others advantages provided by the solution of the invention

The effect of the addition of 0.09%wt. of sodium saccharin and 2% wt. of serine in an injectable solution of lidocaine hydrochloride and epinephrin bitartrate was studied on the chemical stability of the active agents.

The results showed that the addition of 0.09%wt. of sodium saccharin and 2% wt. of serine to an injectable solution of lidocaine hydrochloride and epinephrin bitartrate does not alter the stability of epinephrin content.

Furthermore, the injectable anesthetic solution of the invention fulfils the requirements of the Food and Drug Administration.

Thus, the injectable anesthetic solution of the invention provides a good alternative to the commercially available injectable anesthetic solutions.

## Claims

1. An injectable anesthetic solution comprising:
- an anesthetic agent, preferably selected from lidocaine derivates; tetracaine derivates; xylocaine derivates; mepivacaine derivates; prilocaine derivates; bupivacaine derivates; etidocaine derivates; ropivacaine derivates; articaine derivates; and any combinations thereof;
- optionally, a vasoconstrictor;
- a pharmaceutically acceptable solvent; and
- a bitterness suppressant comprising or consisting of a mixture of at least two among sodium saccharin, sorbitol and an amino acid, wherein the amino acid is selected from: serine, threonine and their mixtures.

2. The injectable anesthetic solution according to claim **1,** wherein the injectable anesthetic solution comprises a vasoconstrictor which is an epinephrin, preferably the vasoconstrictor is epinephrin bitartrate.

3. The injectable anesthetic solution according to claim **1** or claim **2,** wherein the anesthetic agent is a lidocaine or a derivate thereof; preferably is lidocaine hydrochloride.

4. The injectable anesthetic solution according to any one of claims **1** to **3,** wherein the pharmaceutically acceptable solvent is water, preferably selected from sterilized water, purified water and osmosed water.

5. The injectable anesthetic solution according to any one of claims **1** to **4,** wherein the bitterness suppressant consists of sodium saccharin and serine.

6. The injectable anesthetic solution according to any one of claims **1** to **5,** wherein the amount of amino acid ranges from 1% to 6% wt., preferably ranges from 2% to 3% wt., relative to the total weight of the injectable anesthetic solution.

7. The injectable anesthetic solution according to any one of claims **1** to **6,** wherein the amount of sodium saccharin and/or sorbitol, preferably sodium saccharin, ranges from 0.01% to 5% wt., preferably from 0.01% to 1% wt., more preferably is 0.09% relative to the total weight of the injectable anesthetic solution.

8. The injectable anesthetic solution according to any one of claims **1** to **7,** further comprising one or more additives selected from the group consisting of: buffering agents; preservative compounds; stabilizers; contrast media agents; and their mixtures thereof.

9. The injectable anesthetic solution according to claim **8,** wherein the preservative compound is selected from: sodium bisulfites, potassium bisulfites or metabisulfites, ascorbic acid, citric acid, ethylene diamine tetra acetic acid (EDTA), disodium edetate, sodium hydroxide, benzyl alcohol, phenylethyl alcohol, phenol, meta-cresol, chlorobutanol, thimerosal, phenylmercuric salts, and any combinations thereof; preferably is sodium metabisulfite or potassium metabisulfite; preferably the injectable anesthetic solution comprises a preservative compound which is selected from disodium edetate, sodium hydroxide and any mixture thereof.

10. The injectable anesthetic solution according to any one of claims **1** to **9,** comprising:
- from 0.01% wt. to 5% wt., preferably 2.1% wt., of an anesthetic agent which is lidocaine hydrochloride;
- from 0.0001% wt. to 1% wt., preferably 0.002% wt., of a vasoconstrictor which is epinephrin bitartrate;
- a pharmaceutically acceptable solvent; and
- a bitterness suppressant comprising or consisting of:
- from 0.01% to 5% wt., preferably 0.09%, 3% or 3.09%, of a sweetener selected from: sodium saccharin, sorbitol and their mixtures; and
- from 1% to 6% wt., preferably from 2% to 3% wt., more preferably 2.4% or 2.7%, of an amino acid selected from: serine, threonine and their mixtures;
said amounts being relative to the total weight of the injectable anesthetic solution.

11. A method for manufacturing the injectable anesthetic solution according to any one of claims **1** to **10,** comprising mixing:
- an anesthetic agent, preferably which is lidocaine or a derivative thereof;
- optionally, a vasoconstrictor, preferably which is epinephrin bitartrate;
- a pharmaceutically acceptable solvent; and
- a bitterness suppressant comprising or consisting of a mixture of at least two among sodium saccharin, sorbitol and an amino acid, wherein the amino acid is selected from: serine, threonine and their mixtures.

12. The injectable anesthetic solution according to any one of claims **1** to **10,** for use in anesthesia during dentistry, oral surgery and/or maxillofacial surgery.

13. The injectable anesthetic solution for use according to claim **12,** wherein dentistry, oral surgery and/or maxillofacial surgery is filling dental root canals.

14. The injectable anesthetic solution for use according to claim **12,** wherein dentistry, oral surgery and/or maxillofacial surgery is dental crowns positioning and/or root extractions.

15. A pre-filled syringe filled with the injectable anesthetic solution according to any one of claims **1** to **10.**
